# EUROPEAN PATENT SPECIFICATION

(11) **EP 1 134 585 B1**
(45) Date of publication and mention of the grant of the patent: **01.02.2006**
(21) Application number: 01830148.1
(22) Date of filing: 02.03.2001
(51) Int. Cl.: G01N 33/68, G01N 33/53, G01N 33/50, C12Q 1/02

(54) **Biosensor and method for the monitoring of herbicides**
Biosensor und Verfahren zur Überwachung von Herbiziden
Biocapteur et méthode pour contrôler les herbicides

(30) Priority: 03.03.2000 IT RM000112
(43) Date of publication of application: 19.09.2001
(73) Proprietor: CONSIGLIO NAZIONALE DELLE RICERCHE, I-00185 Roma (IT)
(72) Inventor: Giardi, Maria Teresa, 300 Monterotondo, Scalo 00016 - Roma (IT); Leonardi, Chiara, 300 Monterotondo, Scalo 00016 - Roma (IT); Esposito, Dania, Monterotondo, Scalo 00016 - Roma (IT); Margonelli, Andrea, Monterotondo, Scalo 00016 - Roma (IT); Mattoo, Autar, Beltsville, MD 20705-2350 (US); Angelini, Giancarlo, Monterotondo, Scalo 00016 - Roma (IT)
(74) Representative: Capasso, Olga

(56) References cited:
- EP-A- 0 654 662
- R. CARPENTIER, S.LEMIEUX, M. MIMEAULT, M. PURCELL, D.C. GOETZE: "A photoelectrochemical cell using immobilized photosynthetic membranes" BIOELECTROCHEMISTRY AND BIOENERGETICS, vol. 22, 1989, pages 391-401, XP001094315
- C. LORANGER, R. CARPENTIER: "A fast bioassay for phytotoxicity measurements using immobilized photosyntheti membranes" BIOTECHNOLOGY AND BIOENGINEERING, vol. 44, 1994, pages 178-183, XP001084233
- R.ROUILLON, M.SOLE, R.CARPENTIER, J-L MARTY: "Immobilization of thylakoids in polyvinylalcohol for the detection of herbicides" SENSORS AND ACTUATORS, vol. B 26-27, 1995, pages 477-479, XP002206790
- M. NAESSENS, C. TRAN-MINH: "Biosensor using immobilized Chlorella microalgae for determination of volatile organic compounds" SENSORS AND ACTUATORS, vol. B59, 1999, pages 100-102, XP002206791
- D. MERZ, M. GEYER, D.A. MOSS, HJ ACHE: "Chlorophyll fluorescence biosensor for the detection of herbicides" FRESENIUS J ANAL CHEM , vol. 354, 1996, XP002206792
- W. OETTMEIR: "Herbicide resistance and supersensitivity in photosystem II" CMLS CELLULAR AND MOLECULAR LIFE SCIENCES, vol. 55, 1999, pages 1255-1277, XP002206793
- K.H. PASTRIK, U. KÄRST, R.D. SCHMID: "Mutanten von Cyanobakterien mit erhöhter empfindlichkeit gegenüber herbiziden" Z. WASSER-ABWASSER-FORSCH., vol. 24, 1991, pages 12-15, XP001084412
- N. ATAL, P.P. SARADHI, P.MOHANTY: "Inhibition of the Chloroplast photochemical reactions by treatment of wheat seedings with low concentrations of cadmium: analysis of electron transport activities and changes in fluorescence yield" PLANT CELL PHYSIOL. , vol. 32, no. 7, 1991, pages 943-951, XP002206794
- E. FRANCO, S. ALESSANDRELLI, J. MASOJIDEK, A. MARGONELLI, M.T. GIARDI: "Modulation of D1 protein turnover under cadmium and heat stresses monitored by (35 S)methionine incorporation" PLANT SCIENCE, vol. 144, 1999, pages 53-61, XP002206795
- JL MARTY, D. GARCIA, R. ROUILLON: "Biosensors: potential in pesticide detection" TRENDS IN ANALYTICAL CHEMISTRY, vol. 14, no. 7, 1995, pages 329-333, XP002206796

## Description

### Field of the invention

This invention refers to the production and application of a portable system (named "Bioherbi") for the selective environmental monitoring of polluting herbicides in the surface and underground waters.

The system object of this invention is based on an innovative device using as biomediator the protein complex Photosystem II (PS II), isolated from photosynthetic organisms and mutants thereof resistant to subclasses of herbicides, interfaced, by an original technique which is also within the scope of this invention, with a fluorescence transduction system. This protein system, identified as biosensor, in an original way and forming an integral part of this invention, is sensitive to the class of the herbicides acting at level of the photosynthesis and distinguishes the individual subclasses.

### Uses

Monitoring of the herbicide content (total and by chemical compound subclass) in the surface and underground waters, according to CEE and USA directives.

### Advantages

Such a system, as sensor for the herbicides, shows the following advantages:
I) to be portable;
II) to have low production and operating costs;
III) to show a selective sensitivity able to be modulated depending on the method of preparation of the biological sample;
IV) to allow the identification of the class (herein photosynthetic) and the subclasses (for example triazinic or phenylureidic) of the herbicides and quantification thereof;
V) to allow a high number of low cost analyses be carried out, as prescreening, focusing further laboratory analyses;
VI) to be used easily without requiring specifically trained technical people.

### Background of the invention

The invention is based on the principles reported below.

### Biosensor

The basic principle shared by all the sensors consists of their ability to detect an analyte at a very low concentration, due to the interaction occurring between a biological/biochemical system (suitably immobilized) and involved analyte. This interaction results in a response of the biological/biochemical system which is detected (signal transduction) and continuously monitored.

The biological/biochemical matrix can be consisted of enzymes, antibodies, macromolecules, cells or supra-cellular structures. The methods for the transduction of the signal can be electrochemical, optical, acoustic, microgravimetric, microcalorimetric or capacitive. Depending on the combined selection among "biological element - transducer - processing system of the signal" biosensors different with reference to the detection ability and potential utilization are obtained. Indeed not all the combinations are viable or advantageous.

The selection of the biological element determines the selectivity and specificity grade of the biosensor against the analyte, but it is essential also the coupling mode existing between reactive biological layer and signal transducer. In fact as an interaction or reaction between the biological element and analyte occurs, this results in a variation of the chemical-physical parameters identifying the reactive complex in comparison with its resting state. The amount of this variation determines the recognition and quantification of the analyte.

Essential characteristics of biosensors are sensitivity and selectivity which the biological component is able to provide, in connection with the use simplicity and versatility resulting yet from the selected transduction method, usually compatible with the need of miniaturization, low operating cost and continuous monitoring (for information about the matter see Techniques and instrumentation in analytical chemistry Vol. 11, Biosensors. Eds., F Scheller and F Schubert, Elsevier, Amsterdam, London, New York, Tokyo 1992). These characteristics as a whole are particularly interesting in the environmental field for the detection, control and measure of pollutants, for example, within the water bearing layers, river, lake and sea ecosystem and for the control of the observance of the stated limits of the current regulations.

### Photosynthetic herbicides acting on Photosystem II (PS II)

Derivatives of urea, triazines, diazines, phenolic compounds and like represent classes of highly important compounds from an economic point of view in many sectors of the chemical, pharmaceutical and agricultural industries. They constitute the basic products for the chemical weeding of various crops. The compounds belonging to these classes (Ureidic - for example: Diuron, Neburon, Isoproturon, Clortoluron, Linuron, Metobromuron, Cicluron, Metabenzthiazuron, Etidimuro; Triazinic - for example: Simazyn, Ametryn, Desmetryn, Prometryn, Terbutylazine, Terbutryn, Terbumeton, Clanazine, Metribuzin, Metamitron, Exazinone; Diazinic - for example: Bromacil, Lenacil, Cloridazon, Piridate, Bentazone; Phenolic - for example: Bromoxynil and loxynil) represent about 50 % of the herbicides currently used in agriculture, their worldwide consumption amounting to many tons. The compounds of these groups can be applied both in pre- and post-emergency and their activity mechanism results in the inhibition of chlorophyll photosynthesis by blocking the electron flow at level of the Photosystem II (PS II) and consequent interruption of the water photolysis and oxygen formation (Hill reaction). This activity results from the interaction of the herbicide with a protein component of the chloroplast membrane, which performs a primary role in the transport of the electrons from water to NADP (for a test on the matter see Herbicide Handbook of the Weed Society of America. Ed. C J Wilard, Neil Street, Champaign, Illinois. 1994. Residue reviews, Eds FA Gunther and JD Gunther, Springer-Verlag, Berlin, Heidelberg, New York, 1970).

The Phosystem II (PS II) has been isolated from both photosynthetic bacteria and higher plants and it has been already showed to be still able to bind photosynthetic herbicides when it is in an isolated form (MT Giardi, J Barber, MC Giardina and R Bassi, 1990, Z. Naturforsch, 45 366-372. MT Giardi, J Marder and J Barber 1988, Biochim et Biophys acta, 934, 64-71).

Another feature of the herbicides is their good sustained activity in the soil where they are adsorbed by colloids and organic matter. More soluble compounds undergo percolation, mainly in loosed sand soils poor in organic matter. Most part thereof yet is localized within more surface layers of the soil and their persistence therein is variable from middle to high depending on the active principle and pedoclimatic conditions. Therefore these substances or degradation products thereof can both percolate through the soil under the action of the rain and, in solution or adsorbed on soil particles, reach, by means of irrigation canals, the surface and underground water streams.

Wide and prolonged use of certain herbicides, principally atrazine, caused selection and consequent diffusion of herbicide resistant biotypes derived from sensitive infesting species (Weed and crop resistance to herbicides, Eds R De Prado, J Jorrin and L Garcia Torres. Kluwer Academic Publishers, 1997, ISBN 0-7923-4581-9). Although atrazine has been withdrawn from the market, analogous molecules (as simazine differing only by a side-chain methyl group) currently are widely used.

Due to their known high toxicity for humans and animals, the indiscriminate use of herbicides results in severe environmental problems. In fact the problem of the pollution from herbicide of rivers, lakes and underground streams is widely known. For example dinoseb is prohibited in U.S. and many other countries due to its toxicity (US Federal Register, 1986) and atrazine is prohibited too. (US EPA, 1988). CEE, by issuing "European Water Act of 1980" document, stated that the concentration of herbicides in water must be lower than 0,1 mg/l or 0,5 mg/l of individual or total herbicides, respectively.

Therefore it is apparent that in order to detect so low residual concentrations extremely sensitive and reliable analytical methods are required.

### Biomediator

The sensitive core of a biosensor is the biological component thereof (named biomediator) from which the peculiarities of the device depend. In our case the Photosystem II (PS II) is extremely interesting and suitable for the development of a biosensor. In fact when exposed to illumination it shows both fluorescence and development of oxygen and charge transfer, all activities inhibited in the presence of photosynthetic herbicides. The advantage resulting from the use of PS II consists of the particular simplicity of the transduction of the signal correlated to the variation of the biological activity which therefore can be measured directly without the use of any "marker" type or introduction of competitors complicating both the analytical methods and measuring instruments.

The use of PS II for monitoring the herbicides was suggested since 1960 when whole cells of photosynthetic microorganisms were used in laboratory to this goal. However the large scale use of this important biological component was limited by its intrinsic instability impairing the analytical application (C Loranger and R Carpentier, 1994, Biotechnology Bioengineering, 44, 178-183).

A Photochemical cell using thylakoid membranes, containing a PS II, immobilized in an albumin-glutaraldehyde crosslinked matrix has been disclosed (R Carpentier, S Lemieux, M Mimeault, M. Purcell and DC Goetze, 1989, Bioelectrochemistry and Bioernergetics, 22, 391-401). Immobilization of thylakoids in polyvinylalcohol for the detection of herbicides has also been disclosed (R Rouillon, M Sole, R Carpentier and J-L Marty, 1995, Sensors and Actuators, B 26-27, 477-479). Biosensors using immobilized Chlorella microalgae on the membrane of an oxygen electrode for the determination of volatile organic compounds have also been disclosed (M Naessens and C Tran-Minh, 1999, Sensors and Actuators, B 59, 100-102).

Recently remarkable progresses in the improvement of the Photosystem II (PS II) "stabilization" (M Koblizek, J Masojidek, J Komenda, T Kucera, R Pilloton, A Matto, MT Giardi, 1988, Biotechnology and Bioengineering, 60, 664-669) are obtained, principally since we are able to immobilize PS II isolated from thermophilic cyanobacteria. The selection of this particular photosynthetic organism allowed the use of a biomediator which proved stable over many hours at ambient temperature and under measuring conditions (flow cell equipped with a Clark electrode for the measure of the inhibition of the oxygen development in the presence of herbicides), furthermore showing a remarkable sensitivity (for example from 10⁻¹⁰ mol/l for Diuron to 10⁻⁷ mol/l for Bromoxynil). This system also, however, suffered from operating problems: use complexity, long times for standard measure timing, voluminous equipment, etc.

These premises induced us to develop what is the object of this invention. The invention and principal components thereof will be explained in detail below.

### Invention

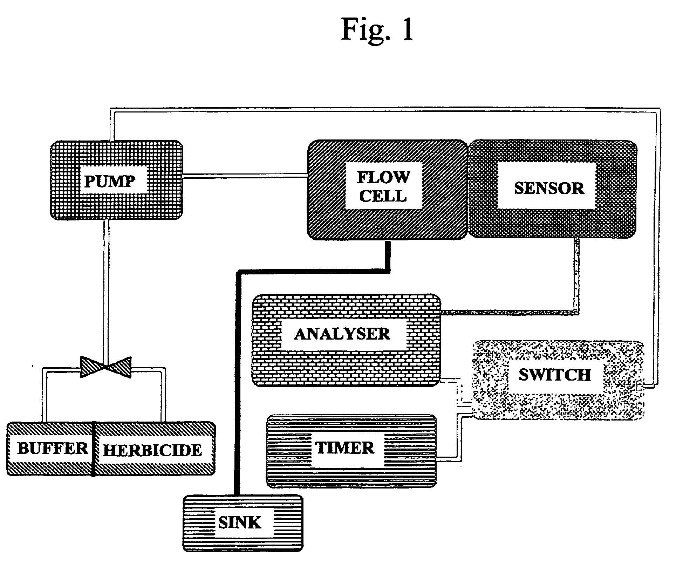

The equipment (see block diagram in Fig. 1) consists of:
1. a flow cell where is inserted a tight and interchangeable block, equipped with filtering septum wherein the biomediator is immobilized;
2. a peristaltic pump promoting the movement of 1) the aqueous sample, containing the herbicide being measured, in the flow cell and then through the biomediator and 2) the biomediator washing and regenerating buffer. The flows are alternated by a three way, suitable valve, also timed;
3. a fluorescence sensor (commercially available device) which provides excitation light and detects the fluorescence signal. The excitation light is emitted by a system of corona arranged LEDs focused on the flow cell and biomediator, which system provides excitation light (650 nm) to the device. The diode system affords the advantages in providing light with reduced heat loss and emitting the signal very quickly. An optical "feedback" circuit controls and adjusts the variations of light intensity emitted and due to variations of external temperature;
4. an optical fiber, fluorescence transducer and closely connected to the flow cell;
5. a fluorescence measuring analyzer, closely connected to the optical fiber;
6. a computer for the control of the system and analysis of the fluorescence data;
7. a timer equipped with 1) a stopped-flow actuator, in order to automatize the fluorescence measures in the flow cell containing the supported biomediator carried out under stopped flow conditions and 2) a delayer-actuator in order to allow the fluorescence sensor to carry out the measure after a prefixed time period after the flow stop;
8. interchangeable blocks of "wild-type" biomediator and immobilized "mutant";
9. container of the biomediator washing and regenerating buffer.
This equipment was assembled, as a whole, in a portable structure in order to provide a versatile and easy use thereof in actual operating conditions, lowering both the operating and constructing cost.

The biosensor, as a whole, shows the advantage, in comparison to previous systems based on the Clark electrode (M Koblizek, J Masojidek, J Komenda, T Kucera, R Pilloton, A Mattoo, MT Giardi, 1998, Biotechnology and Bioengineering, 60, 664-669), to be, in addition to portable, ready to use; in comparison to the biosensors based on the fluorescence of the chlorophyll (D Merz, M Geyer, M Moss, DA Anche, 1996, Fresenius J Analytical Chemistry 354, 299-305) to provide a measure of the inhibition times and/or fluorescence integrated areas instead of peak measures, clearly improving the sensitivity and precision thereof. Furthermore our biosensor, as described more completely below, has the original feature, with respect to all the previous systems (for a review see JL Marty, D Garcia, R Rouillon, Trends in Analytical Chemistry, 14, 329-332), of being able to distinguish the subclasses of the photosynthetic herbicides by using specific immobilized biomediators isolated from mutated organisms. Such mutated organisms prove to be "resistant" to one subclass of photosynthetic herbicides but not to the others (for example a mutant is resistant to triazines whereas it is sensitive to ureas, with different fluorescence emissions, see D Lazar, 1999, Biochim Biophys Acta, 1.28). Therefore a "wild-type" organism is sensitive to triazines whereas the corresponding mutated is not. Therefore it follows that in the first case a response with fluorescence variation associated to a "non response" for the mutated organisms certainly identifies the subclass.

### Biomediator

The realization of such a biosensor involved the preparation and isolation of PS II "particles" in non ionic detergents and their chemical-physical and structural characterization (MT Giardi, 1993, Planta, 190, 107-113). PS II particles are obtained both from higher plants (stability limit to +5°C) and thermophilic cyanobacteria (the latter being stable to relatively high temperatures: +50 - +60°C°). In order to improve the stability of the biomediator we used the adaptation capacity of the photosynthetic organisms to high temperatures whereby they were grown at relatively high temperatures (33°-35°C) or subjected over 48 hours to a moderate thermal stress (33°-40°C) (E Franco, S Alessandrelli, J Masojidek, A Margonelli and MT Giardi, 1999, Palnt Science, 53-61).

Furthermore to optimize the orientation and cohesion of the biomediator molecules both the most important techniques for immobilization of proteins on optical surfaces (covalent bonds, physical adsorption, ecc.) and the protein filming by cross-linking were examined (for example, on gold surface) (T Wink, SJ van Zuilen, A Bult and WP van Bennekom, 1997, Analyst, 122,43-50).

Experimentally it has been noted that the technique of PS II immobilization on surfaces by chemical cross-linking (BSA-GA) involves both activity loss as fluorescence response and sensitivity loss against the herbicide. Furthermore it has been noted that the technique for the physical entrapment of PS II particles in membranes, although without appreciable activity losses, resulted in problems for both the permeation of the herbicide and subsequent regenerative washing step.

Therefore an innovative and original methodology for the cross-linking and entrapment of the biomediator was set up. This include a treatment with a CdCl₂ solution which causes the cross-linking of the SH groups of the Photosystem II proteins, resulting in a network more compact and resistant to the bleeding activity of the herbicide containing effluent, and in addition a higher stability of the biomediator and increase of the sensitivity against the herbicide: the sensitivity against the herbicide is on the order of 10-100 times higher than when immobilized with CdCl₂.

The thus treated biomediator is further immobilized on a porous septum constituted of glass material having a thickness form 0,5 mm to 6,0 mm and a pore size from G1 to G5, as a component of the measuring flow cell.

To further increase the stability of the biomediator the usefulness of a system for the refrigeration of the solution containing herbicide forwarded into the measuring cell experimentally was observed. Below is reported a brief description of the best operating conditions according to which the best responses from PS II isolated from a) higher plants treated at high temperature (33°C - 40°C) over several days and incubated with a CdCl₂ solution (from 0,2 mM to 10 mM) over several hours and b) thermophilic cyanobacteria grown at temperatures higher than 50°C and treated also with CdCl₂ and refrigerating system at 5°C, with a flow of herbicide solution from 0,2 ml/m to 2,0 ml/m are obtained. Under such conditions, for example, for Atrazine, Simazine and Diuron are obtained sensitivities at concentrations lower than nanomolar, whereas the sensitivity values for Bromoxynil and loxynil are one order of magnitude higher. In any case the sensitivity results are dependent on the washing times of the biomediator with the solution containing the herbicide whereby it is possible to reach sensitivity values on the order of 10⁻¹⁰ M, prolonging, however, the running time of each analysis beyond 60 minutes.

The stability of the biomediator in operating conditions can be defined as the time required to have a 50 % reduction of the activity, which in our case is 48 hours. By means of a suitable washing and regenerating system (Tricine buffer, 50 mM, pH = 7, containing NaCl, 1,0 - 5,0 mM, MgCl₂ 5,0 - 20 mM and CdCl₂ 0,2 - 5,0 mM) reproducible measures over two days using a single body containing the biomediator regenerated after each measure can be obtained.

### Specificity of the biomediator

In order to have a signal specific for an individual class of herbicides the peculiar properties of field selected mutants of Atrazine resistant higher plants are utilized (for example: *Senecio vulgaris, Poa vulgaris, Amaranthus retroflexus,* ecc.). Such an approach was pursued because it is known that the mutation of a single amino acid on D1 protein (produced by psbA gene) is able to induce resistance to the herbicide (for example the replacement of a serine for a glycine induces "resistance" to Atrazine but not to other herbicides) (W Oettmeier, 1999, CMLS, 55, 1255-1277). The biomediator, consisting of isolated PS II, immobilized as before described, is resistant to the class of triazinic compounds and therefore selective for the classes of herbicides other than triazine (therefore sensitive to phenolic, ureidic, diazinic herbicides). Resistant mutants, sensitive to different classes of herbicides, are available for research purposes (W Oettmeier, 1999, CMLS, 55, 1255-1277), whereby it is possible to obtain a wide multiplicity and sensitivity from a serial use of such biomediators. Hypersensitive mutants were already used to increase the sensitivity of the biomediator (KH Pastrik, U Kaerst, RD Schmid, D Rolf, 1991, Abt Enzymtechnol., Ges. Biotechnol. Forsch., Braunschweig, Fed. Rep. Ger. Z. Wasser Abwasser Forsch. 24(1), 12-15).

A list of species of cyanobacteria and algae suitable to be used in the invention, available moreover from the CSMAICNR (Centro Studi Microorganismi Autotrofi, Via Ponte di Formicola, 72 I-50018 Scandicci (FI) Italy) centre, follows.

**Table I. Prospect of species of cyanobacteria and algae**

| Name | Division | | Family | Species | Optimum growth temperature |
|---|---|---|---|---|---|
| | | | Oocystaceae | *Chlorella* | 35°c |
| Green | Chlorophyta | Chlorococcales | | *Sorokiniana* | |
| algae | | | | | |
| | | | | *Chlorella* | 25°-35°C |
| | | | | *Zofinngiensis* | |
| | | | Chlorococcacea | *Spongiochloris* | 30°C |
| | | | and | *Typea* | |
| | | | | *Scenedesmus* | 30°C |
| | | Ulotrichales | Scenedesmacea | *quadricauda* | |
| | | | and | | |
| | | | | *Koliella sp.* | 15°-20°C |
| | | | Ulotrichaceae | | |
| - | Eustigmatophyt | Monodus | | *Pleurochloris* | 15°-20°C |
| | a | subterraneus | | *meringensis* | |
| Green | Cyanophyta | Oscillatoriales | Oscillatoriaceae | *Spirulina* | 35°C |
| blue | (Cyanobacteria) | | | *platensis* | |
| algae | | | | | |
| | | Chroococcales | Chroococcaceae | *Synechcoccus* | 60°-63°C |
| | | | | *elongatus* | |

To obtain higher stability of the biomediator particles, mutants of thermophilic cyanobacteria obtained by the "Side-Directed Mutagenesis" technique can be used (W Oettmeier, 1999, CMLS, 55, 1255-1277).

As for this patent both mutant types (from higher plants and cyanbacteria) in comparison with their "wilt-type" species were used.

A not limiting list of the mutant strains used in the present invention follows.

### Mutants of herbicide resistant photosynthetic bacteria

### Individual Mutants

| Mutation | *Organism* | Code | Resistance R/S | Supersensitivity R/S |
|---|---|---|---|---|
| Gly192->Asp | *Rubrivivax* | | Atrazine 6 | |
| | *Gelatinosus* | | o-Phenanthroline 3 | |
| | | | Terbutryn 300 | |
| G1U212->LYS | *R.viridis* | MAV2 | Atrazine | |
| Phe216->Leu | *-T-capsulatus* | | Ametryn 3,1 | |
| | | | Atrazine 4,2 | |
| | | | Diuron 1 | |
| | | | Prometon 1,8 | |
| | | | Prometryn 1,4 | |
| PhC2,6―Pro | *R. capsulatus* | | Ametryn 1,9 | |
| | | | Atrazine 1,9 | |
| | | | patron 1 | |
| | | | Prometon 1,8 | |
| Phe216->Thr | *R.capsulatus* | | Atrazine 3,5 | |
| | | | Diuron 1 | |
| Phe216→Val | *R.capsulatus* | | Atrazine 1,8 | |
| | | | Diuron 1 | |
| -Phe216→Ser | *R.-viridis* | T6 | o-Phenanthroline 333 | |
| | | | Terbutryn >2800 | |
| -Pha216->Ser | *R-viridis* | MAV3 | Atrazine | |
| Tyr222->Phe | *R.-viridis* | T4 | Ametryn 330 | |
| | | | Atrazine >600 | Diuron <0,0001 |
| | | | Desmetryn 50 | Iloxynil <0,01 |
| | | | o-Phenanthroline 10 | |
| | | | Terbutryn 660 (>5000) | |
| Tyr222->Gly | *R. sphaeroides* | YG222 | o-Phenanthroline 26 | |
| | | | Terbutryn >3000 | |
| Ser223->Pro | *R. sphaeroides* | SP223 | o-Phenanthroline 6,25 | |
| | | | Terbutryn >3000 | |
| Thr226->Ala | *R. capsulatus* | | Ametryn 4 | o-Phenanthro- |
| | | | | line 0,03 |
| | | | Atrazin 3,1 | Terbutryn 0,6(?) |
| | | | Diuron 1 | |
| | | | Prometon 1,8 | |
| | | | Prometryn 1,4 | |
| | | | Terbutryn 1,7 | |
| Thr226->Met | capsulatus | | Ametryn 2,5 | |
| | | | Atrazine 3,1 | |
| | | | Diuron 1 | |
| | | | Prometryn 1,1 | |
| | | | Terbutryn 1,7 | |
| Gly228->Arg | *R. capsulatus* | | Ametryn 4 | |
| | | | Atrazine 5,4 | |
| | | | Diuron 1 | |
| | | | Prometon 1,2 | |
| | | | Prometryn 1,1 | |
| | | | Terbutryn 1,3 | |
| Gly228->Val | *R. capsulatus* | | Ametryn 5,3 | |
| | | | Atrazine 4,2 | |
| | | | Diuron 1 | |
| | | | Prometon 1,3 | |
| | | | Prometryn 1,4 | |
| | | | Terbutryn 1,5 | |
| I1e229->Ala | *R. capsulatus* | | Atrazine 1,8 | |
| | | | Diuron 1 | |
| | | | Prometron 1,2 | |
| I1e229->Cys | *R. capsulatus* | | Atrazine 2 | |
| | | | Diuron 1 | |
| I1e228->Leu | *R. capsulatus* | | Ametryn 2,5 | |
| | | | Atrazine 3,5 | |
| | | | Diuron 1 | |
| I1e229→Met | *R. capsulatus* | | Ametryn 5,3 | |
| | | | Atrazine 5,4 | |
| | | | Diuron 1 | |
| | | | Prometron 1,2 | |
| | | | Prometryn 2 | |
| | | | Terbutryn 1,9 | |
| Ile229->Met | *R. shaeroides* | | Atrazine >7 | |
| Ile229->Met~.Met | *R.shaeroides* | M29 | o-Phenanthro me 11 | |
| | | | Terbutryn 120 | |
| Ile229->Ser | *R, capsulatus* | | Atrazine 1,8 | |
| Ile229->Ser | *R. capsulatus* | | o-Phenanthroline 1,3 | Terbutryn 0,4 |
| Ile229->Thr | *R. capsulatus* | | Ametryn 1,9 | |
| | | | Atrazine 3,7 | |
| | | | Prometon 1,2 | |
| | | | Prometryn 1,4 | |
| GluM34->Lys | *R. rubrum* | M6 | o-Phenanthroline >25 | |
| | | | Terbutryn > 125 | |
| | | | NH-Thiazoles (see Text) | |

| Double Mutants | | | | |
|---|---|---|---|---|
| Phe216→Ser | *R viridis* | T3 | o-Phenathroline 67 | |
| ValM263→Phe | | | Terbutryn 166 | |
| Arg217→His | *R. midis* | MAV4 | Atrazine | |
| Val224→Leu | | MAV5 | | |
| Arg217→His | *R. viridis* | | o-Phenanthroline 5 | |
| Ser223→Ala | | | Terbutryn 222 | |

Mutations in D1 protein, reaction core of the Photosystem II which gives herbicide resistance and/or supersensitivity.

### Single Mutant

| Mutation | Organism | Code | R/S Resistance | Supersensitivity R/S |
|---|---|---|---|---|
| Phe211->Ser | *Synechococcus sp. PC7002* | | Atrazine 7 | |
| | | | Diuron 2 | |
| | *Synechocystis* PCC6714 | Azl | Atrazine 10 | |
| | | | Metribuzin 8 | |
| Val219->Ile | *Chlamydonionas* | Dr2 | Atrazine 2 | Ketonitrile 0.6 |
| | | | Benzthiazuron 16 | Lenacil 0,8 |
| | | | BNT 2 | |
| | | | Bromanil 1,2-2 | |
| | | | Cyanoacrylate 2 | |
| | | | Dinoseb 7,5 | |
| | | | Diuron 15-32 | |
| | | | loxynil 50 | |
| | | | Metabenzthiazu- | |
| | | | ron 62 | |
| | | | Metamitron 1.2 | |
| | | | Metribuzin 200 | |
| | | | Triazinone | Triazinone |
| | *Synechococcus sp. PCC7002* | | Atrazine 2 | |
| | | | Diuron 10 | |
| Tyr237->Phe | *Synechocystis sp. PC6803* | | Atrazine 2 | BNT 0,2 |
| | | | Diuron 5 | |
| | | | loxynil 5 | |
| Lysa)238->Val | *Synechocystis sp.* PC6803 | | Diuron 1,3 | Atrazine 0,6 |
| | | | loxynil 2,3 | BNT 0,4 |
| Ile248->Thr | *Synechocystis PCC6714* | M30 | Diuron 2 | |
| | | | Metribuzin 28 | |
| Ala250->Arg | *Chlamydomonas* | | Metamitron 2,5 | Atrazin 0.25 |
| | | | Phenmedipham | Bromoxynil 0,3 |
| | | | 6,3 | |
| | | | Terbutryn 1,3 | loxynil 0,5 |
| | | | | Metribuzin 0,5 |
| Ala250->Asn | *Chlamydomonas* | | loxynil 4 | Atrazine 0,3 |
| | | | Metamitron 8 | Bromoxynil 0,2 |
| | | | Phenmedipham 4 | Metribuzin 0,8 |
| | | | Terbutryn 1,3 | |
| Ala250->Asp | *Chlamydomonas* | | Metamitron 5 | Atrazine 0,25 |
| | | | Metibuzin 1,3 | Bromoxynil 0,16 |
| | | | Phenmedipham 5 | loxynil 0,2 |
| | | | | Terbutryn 0,4 |
| A1a250->His | *Chlamydomonas* | | Atrazine 2,8 | Bromoxynil 0,16 |
| | | | loxynil 1 | |
| | | | Metamitron 8 | |
| | | | Metribuzin 1,3 | |
| | | | Phenmedipham 10 | |
| | | | Terbutryn 1,8 | |
| A1a250→IIe | *Chlamydomonas* | | loxynil 1 | Atrazine 0,5 |
| | | | Metamitron 1 | Bromoxynil 0,3 |
| | | | Phenmedipham | Metribuzin 0.25 |
| | | | 2,5 | Terbutryn 0,5 |
| A1a250->Tyr | *Chlamydomonas* | | Metamitron 2 | Atrazine 0,4 |
| | | | Phenmedipham 20 | Bromoxynil 0,4 |
| | | | Terbutryn 1,3 | loxynil 0,8 |
| | | | | Metribuzin 0.6 |
| A1a251->Cys | *Chlamydomonas* | | Bromoxynil 6,3 | Atrazin 0,8 |
| | | | loxynil 10 | Phenmedipham |
| | | | Metamitron 6,3 | 0,8 |
| | | | Metribuzin 2,5 | |
| | | | Terbutryn 1,0 | |
| A1a251→Gly | *Chlamydomonas* | | Atrazin 1 | Metribuzin 0,6 |
| | | | Bromoxynil 2,5 | |
| | | | loxynil 4 | |
| | | | Metamitron 10 | |
| | | | Phenmedipham 25 | |
| | | | Terbutryn 1 | |
| A1a250->His | *Chlamydomonas* | | Atrazine 2,8 | Bromoxynil 0,16 |
| | | | loxynil 1 | |
| | | | Metamitron 8 | |
| | | | Metribuzin 1,3 | |
| | | | Phenmedipham 10 | |
| | | | Terbutryn 1,8 | |
| A1a250->Ile | *Chlamydomonas* | | loxynil 1 | Atrazine 0,5 |
| | | | Metamitron 1 | Bromoxynil 0,3 |
| | | | Phenmedipham | Metribuzin 0,25 |
| | | | 2,5 | Terbutryn 0,5 |
| A1a250->Tyr | *Chlamydomonas* | | Metamitron 2 | Atrazine 0,4 |
| | | | Phenmedipham | Bromoxynil 0,4 |
| | | | 20 | loxynil 0,8 |
| | | | Terbutryn 1,3 | Metribuzin 0,6 |
| A1a251->Cys | *Chlamydomonas* | | Bromoxynil 6,3 | Atrazin 0,8 |
| | | | loxynil 10 | Phenmedipham |
| | | | Metamitron 6,3 | 0,8 |
| | | | Metribuzin 2,5 | |
| | | | Terbutryn 1,0 | |
| A1a251→Gly | *Chlamydomonas* | | Atrazin 1 | Metribuzin 0,6 |
| | | | Bromoxynil 2,5 | |
| | | | loxynil 4 | |
| | | | Metamitron 10 | |
| | | | Phenmedipham 25 | |
| | | | Terbutryn 1 | |
| A/a251->lIe | *Chlamydomonas* | | Atrazine 2 | Diuron 0,6 |
| | | | Bromacil 11 | |
| | | | Metribuzin 55 | |
| Ala251→Leu | *Chlamydomonas* | | Atrazine 6 | |
| | | | Bromacil 26 | |
| | | | Diuron 4 | |
| | | | Metribuzin 108 | |
| Ala251-Val | *Chlamydomonas* | MZ2 | Atrazine 25 | Ketonitrile 0,5 |
| | | | Benzthiazuron 16 | |
| | | | BNT 8 | |
| | | | Cyanoacrylate 18 | |
| | | | Dinoseb 3 | |
| | | | Diuron 5-8 | |
| | | | DNSJ 1,6 | |
| | | | loxynil 25-40 | |
| | | | Lenacil 160 | |
| | | | Metamitron 124 | |
| | | | Metabenzthiazuron | 126 |
| | | | Metribuzin 1000 | |
| | | | Triazinone | Triazinone |
| | *Synechocystis PCC6714* | M35 | Atrazine 23 | |
| | | | Diuron 3 | |
| | | | loxynil 10 | |
| | | | Metribuzin 200 | |
| *Phe255->Tyr* | *Chlamydomonas* | Ar207+ | Atrazine 15 | Benzthiazuron |
| | | | Cyanoacrylate 39 | 0,3 |
| | | | Dinoseb 3 | Bromacil 0,9 |
| | | | loxynil 2,5 | BNT 0,6 |
| | | | | Diuron 0,6-0,8 |
| | | | | Ketonitrile 0,6 |
| | | | | Metabenzthia- |
| | | | | zuron 0,3 |
| | | | | Metamitron 0,3 |
| | | | | Metribuzin 0,6 |
| | | | Triazinone | Triazinone |
| | | | (see text) | |
| | *Synachococcus PCC7942* | TyrS | Atrazine 25 | Metribuzin 0,8 |
| | | | Diuron 1,5 | |
| | | | loxynil 4 | |
| Gly256->Asp | *Chlamydomonas* | BR24 | Atrazine 15 | |
| | | | Bromacil 10 | |
| | | | Diuron 3 | |
| Arg257->Val | *Synechocistis sp. PCC6803* | | Atrazine 30 | BNT 0,3 |
| | | | Diuron 38 | loxynil 0,8 |
| A1a263->Pro | *Synechocistis sp. PCC6803* | | Atrazine 2000 | |
| | | | Diuron 60 | |
| | | | loxynil 1 | |
| | | | Metribuzin 1600 | |
| | | | Terbutryn 160 | |
| Ser264->Ala | *Chlamydomonas* | MZ1 | Atrazine 125-500 | BNT 0,3 |
| | | MZ3 | Benzthiazuron | i-Dinoseb 0,7 |
| | | | 49-80 | |
| | | MZ5 | Bromacil 106 | DNSJ 0.3 |
| | | | Cyanoacrylate 30 | Hydroxyquino- |
| | | | | line 0,5 |
| | | | Diuron 200 | |
| | | | loxynil 1,3 | loxynil 0,5 |
| | | | Metamitron 30 | Ketonitrile 0,6 |
| | | | Methabenzthia- | |
| | | | zuron 25 | |
| | | | Metribuziin 5000- | |
| | | | 1000 | |
| | | | | Triazinones |
| | | | Phenisopham 40 | |
| | | | Triazinones | |
| Ser265(264) | *Euglena* | ZR | Atrazine 2,3 | |
| ->Ala | | | | |
| | | | Chloroxuron 480 | |
| | | | Diuron 270 | |
| | | | Neburon 2,3 | |
| | | | o-Phenathroline 5 | |
| | | | Siduron 2,5 | |
| Ser264->Ala | *Anacystis nidulans R2* | | Atrazine 10 | |
| | | | Diuron 100 | |
| | | | HQNO 6 | |
| Ser264->Ala | *Synechocistis sp. PCC6714* | DCMU | Atrazine 70 | |
| | | IIA | Diuron 500 | |
| | | | Metribuzin >3000 | |
| Ser264->Ala | *Synechococcus sp. PCC7942* | Dil | Bromacil 33 | loxynil 0,4 |
| | | | Metamitron 3,3 | |
| | | | Metribuzin 5000 | |
| | | | 6 Triazines 3-60 | |
| | | | 7 Ureas 10-1000 | |
| Ser264->Ala | *Synechocistis sp. PCC6803* | | Atrazine 30 | BNT 0,1 |
| | | | Diuron 207 | |
| | | | loxynil 1,2 | |
| Ser264->Gly | *Amarenthus hybridus* | | Ametryn 500 | Bentazon 0,2 |
| | | | Atrazine 966-1033 | Bromnithro- |
| | | | Atraton 100 | thymol 0,6 |
| | | | Bromacil 20 | Diuron 0,1- |
| | | | | 0,13 |
| | | | Chloroxuron 3 | DNOC 0,8 |
| | | | Diuron 1,5 | |
| | | | Fenuron 1,1-1,4 | |
| | | | Lenadi 1880-2040 | |
| | | | Phenmedipham 1266- | |
| | | | 2040 | |
| Ser264→Gly | *Amaranthus retroflexus* | | Ametryn 460 | Bentazon 0,6 |
| | | | Atraton 1000 | Bromnitrothy- |
| | | | | mol 0.22-2 |
| | | | Bromacil 20-> | |
| | | | 2000 | i-Dinoseb 0,5 |
| | | | Chloroxuron 794 | DNOC 0,14-0,5 |
| | | | Diuron 1,0-4 | Fluometuron |
| | | | | 0,25 |
| | | | Fenuron 1, 1-1,4 | loxynil 0,64-1,6 |
| | | | Lenacil 50-590 | Picric acid 0,3 |
| | | | Linuron >3100 | |
| | | | Metribuzin 260->1500 | |
| | | | Metamitron 40 | |
| | | | Neburon 501 | |
| | | | Phenmedipham | |
| | | | 6,3, 1033-1100 | |
| Ser264->Gly | *Amaranthus bouchoniil* | | Atrazine 857 | |
| | | | Diuron 809 | |
| | | | Fenuron 13,5 | |
| | | | Lanacil 4,4 | |
| | | | Phenmedipham 3,8 | |
| Set264->Gly | *Brassica camptestrsl* | | Ametryn 600 | |
| | | | Atraton 800 | |
| | | | Atrazine 600 | |
| | | | Bromacil 40 | |
| | | | Diuron 2,5 | |
| Ser284->Gly | *Chenopodium album* | | Ametryn 400 | Bentazon 0,5 |
| | | | Atraton 100 | |
| | | | Atrazine 100->3000 | |
| | | | Bromacil 30 | |
| | | | Chloruxuron 8 | |
| | | | Diuron 1,3-2 | |
| | | | Fenuron 1692-2115 | |
| | | | Lenacil 13,5-14,1 | |
| | | | Metribuzin 500 | |
| | | | Phenmedipham | |
| | | | 5,0-5,9 | |
| Ser264->Gly | *Phalaris paradoxa* | | Triazines 2-11 | |
| | | | Ureas 62-1000 | |
| Ser264->Gly | *Poa annua* | | Atrazine | |
| Ser->Gly | *Senecio vulgaris* | | Bromacil 100 | |
| | | | Diuron 1,7 | |
| | | | Triazines 250-1000 | |
| Ser264->Gly | *Solanum Iligrum* | | Atrazine >1000 | |
| Ser264->Gly | *Synechococcus PCC7942* | G264 | 6-Triazines 4-50 | 0,4 |
| | | | 7 Ureas (45-1000) | |
| | | | Metribuzin 1500 | |
| Ser264->Asn | *Nicotiana plumbaginifolia* | | Terbutryn | |
| Ser264->Pro | *Synechocystis sp PCC6803* | | Atrazine 10000 | |
| | | | Diuron 1 | |
| | | | loxynil 5 | |
| | | | Metribuzin 25 | |
| | | | Terbutryn 300 | |
| Ser264->Thr | *Nicotiana tabacum* (cell | | Bromacil 64 | Dinoseb 0,13 |
| | culture) | | | |
| | | | Metribuzin 290 | |
| | | | 11 Phenylureas | |
| | | | 6,3-200 | |
| | | | 11 Triazines 47-430 | |
| Ser264->Thr | *Euglena* | MSI | Atrazine 63 | BNT 0,3 |
| | | | Berrzthiazuron 20 | loxynil 0,2 |
| | | | Cyanoacrylate 50 | Ketonittril 0,8 |
| | | | Diuron 20 | |
| | | | Metribuzin 63 | |
| Ser264->Thr | *Solanum tuberosum* (cell | | Atrazine 65 | |
| | culture) | | | |
| Asn266->Asp | *Synechocystis PCC6714* | loxlla | Atrazine 1 | Diuron 0,7 |
| | | | loxynil 2,5 | |
| Asn266->Thr | *Synechocystis PCC6714* | loxl | Atrazine 1 | |
| | | | Bromoxynil 15 | |
| | | | loxynil 9 | |
| Ser268->Pro | *Glycine max* (cell culture) | STR7 | Atrazine 50 | |
| | | | Diuron 3 | |
| Arg263->Gly | *Chlamydomonas* | | Terbutryn 8 | |
| Leu275->Phe | *Chlamydomonas* | Br202 | Atrazine 1 | |
| | | | Bromacil 4,5 | |
| | | | Diuron 5 | |
| | | MZ4 | Atrazine 1 | Cyanoacrylate |
| | | | Benzthiazuron 4 | 0,5 |
| | | | BNT 1,5 | locynil 0,2 |
| | | | i-Dinoseb 3 | |
| | | | Diuron 5 | |
| | | | Ketonitrite 1,3 | |
| | | | Lenacil 3 | |
| | | | Metabenzthiazuron | |
| | | | 1,2 | |
| | | | Metamitron 63 | |
| | | | Metribuzin 20-26 | |
| | | | Phenmedipham | 1 |
| | | | 1,5 | |

| Double Mutants | | | | |
|---|---|---|---|---|
| Phe211→Ala | *Chlamydomonas* | | Atrazine 3 | Diuron 0,8 |
| Met214→Thr | | | Cyanoacrylate 6 | |
| | | | loxynil 3 | |
| | | | Metamitron 2 | |
| | | | Metribuzin 1 | |
| | | | Phenmedipham 25 | |
| Phe211→Gly | *Chlamydomonas* | | Atrazine 6,3 | loxynil 0,8 |
| Met214→Gln | | | Cyanoacrylate 5 | Metamitron |
| | | | Diuron 2 | 0,4 |
| | | | Metribuzin 1 | |
| | | | Phenmedipham 4 | |
| Phe211-+Gly | *Chlamydomonas* | | Atrazine 4 | |
| Met214->Ser | | | Cyanoacrylate 6 | |
| | | | Diuron 5 | |
| | | | loxynil 1,6 | |
| | | | Metamitron 8 | |
| | | | Metribuzin 1,6 | |
| | | | Phenmedipham 79 | |
| Phe211->Ile | *Chlamydomonas* | | Atrazine 2 | Metribuzin |
| Met214->Gly | | | Cyanoacrylate 25 | 0,5 |
| | | | Diuron 5 | |
| | | | loxynil 1,3 | |
| | | | Metamitron 1 | |
| | | | Phenmedipham 79 | |
| Phe211->Ser | *Synechocystis sp. PCC6714* | AzV | Atrazine 100 | |
| Ala251->Val | | | Diuron 3 | |
| | | | loxynil 20 | |
| | | | Metnbuzin 4000 | |
| Phe211->Thr | *Chlamydomonas* | | Atrazine 4 | |
| Met214->Gly | | | Cyanoacrylate 13 | |
| | | | Diuron 10 | |
| | | | loxynil 1,3 | |
| | | | Metamitron 2 | |
| | | | Metribuzin 1 | |
| | | | Phenmedipham 63 | |
| Leu219(218)- | *Euglena* | ZR250 | Diuron 4226 | |
| >Phe | | +C) | | |
| Ser265(264)- | | Zr250 | Atrazine 63 | |
| >Ala | | -C) | | |
| | | | Diuron 1922 | |
| | | Zr480 | Diuron 4045 | |
| | | +C) | | |
| | | Zr480 | Atrazine 71 | |
| | | -C) | | |
| | | | Diuron 1735 | |
| Val219->Ile | *Chenopodium* | LA, L6 | Atrazin 4-14 | |
| A1a251->Thr | *rubrum* (cell culture) | L7 | BNT 0.6-11 | |
| | | | Bromacil 2-25 | |
| | | | Dinoseb 4-8 | |
| | | | Diuron 3-13 | |
| | | | Metribuzin 6-794 | |
| | | | Phanmedipham 2-3 | |
| | | | Propanil 2-71 | |
| Val219->lIe | *Chenopodium rubrum* | LI, L8 | Atrazine 8-9 | |
| la251->Val | (cell culture) | | BNT 25 | |
| | | | Bromacil 15-20 | |
| | | | Diuron 2-3 | |
| | | | Metribuzin 1259/5012 | |
| | | | Phenmedipham 1,5 | |
| | | | Propanil 14-15 | |
| Set221→Leu | *Synechocystis sp.PCC6803* | | Atrazine 14 | |
| Ser222→A1a | | | BNT 2 | |
| | | | Diuron 560 | |
| | | | loxynil 6,2 | |
| Thr227→Ala | *Synechocystis sp. PC6803* | | Atrazine 3 | |
| Thr228→Ala | | | BNT 2,4 | |
| | | | Diuron 14 | |
| | | | loxynil 3 | |
| Ala250→Ser | *Chlamydomonas* | | Metamitron 1,6 | Atrazine 0,5 |
| Phe255→IIe | | | Phenmedipham 10 | Bromoxynil |
| | | | | 0,2 |
| | | | | loxynil 0,2 |
| | | | | Metribuzin |
| | | | | 0,5 |
| | | | | Terbutryn |
| | | | | 0,9 |
| Phe255->Leu | *Synechococcus PCC7942* | Di22 | Atrazine 2.6 | |
| Ser264→Ala | | | Bromacil 37 | |
| | | | Diuron 2647 | |
| | | | Ethidimuron 16 | |
| | | | loxynil 5 | |
| | | | Metamitron 40 | |
| | | | Metribuzin 175 | |
| | | | Terbutryn 1,5 | |
| | | | Thebuthiron 6,2 | |
| | *Synechocystis PCC6714* | DCM | Atrazine 1 | |
| | | Ullb | Diuron 600 | |
| Phe255->Tyr | *Synechococcus PCC 7942* | D5 | Bromacil 17 | Bromoxynil |
| | | | | 0,8 |
| Ser264->Ala | | | S-Cyanoacrylate | Dinoseb |
| | | | 250 | 0,08 |
| | | | R-Cyanoacrylate 13 | loxynil 0,5 |
| | | | Metramitron 2 | |
| | | | Metribuzin 2000 | |
| | | | Phenmedipham 2000 | |
| | | | 6 Triazines 83-660 | |
| | | | 7 Ureas 2,5-250 | |
| Phe255->Tyr | *Synechococcus PCC7942* | TG | Triazines | |
| Ser264→Ala | | | | |
| Ser264→Ala | *Chlamydomonas* | | Atrazine 40 | BNT 0,16 |
| Asn266→Thr | | | Benzthiazuron 50 | loxynil 0,8 |
| | | | Cyanoacrylate 130 | Ketonitrile |
| | | | Diuron 80 | 0,4 |
| | | | Metamitron 80 | |
| | | | Metribuzin 1580 | |
| | | | Phenmedipham 1000 | |

| Triple mutants | | | | |
|---|---|---|---|---|
| Phe211->Val | *Bumilleriopsis* | | Diuron | |
| Val218->Phe | *filiformis* | | | |
| Val219->Ile | | | | |
| Val219->Ile | *Chenopodium rubrum* | L5 | Atrazine 8 | BNT 0,6 |
| Thr220->Ala | (cell culture) | | Bromacil 2 | Dinoseb 0,7 |
| Ser270->Tyr | | | Diuron 2 | |
| | | | Metribuzin 25 | |
| | | | Phenmedipham 2 | |
| | | | Propanil 13 | |
| Val219->Ile | *Chenopodium rubrum* | L3 | Atrazine 14 | BNT 0,6 |
| Thr220->Ala | (cell culture) | | Bromacil 5 | |
| Ser270->Phe | | | Dinoseb 1,1 | |
| | | | Diuron 3 | |
| | | | Metribuzin 25 | |
| | | | Phenmedipham 3 | |
| | | | Propanil 2 | |
| Val219->Ile | *Chenopodium rubrum* | L2 | Atrazine 14 | |
| A1a251->Val | (cell culture) | | BNT 5 | |
| Asn266->Thr | | | Bromacil 120 | |
| | | | Dinoseb 10 | |
| | | | Diuron 3 | |
| | | | Metribuzin 251 | |
| | | | Phenmedipham 9 | |
| | | | Propanil 2 | |
| I1e259->Ser | *Chlamydomonas* | | Atrazine 20 | BNT 0,1 |
| Ser264->Ala | | | Benzthiazuron 20 | loxynil 0,13 |
| Asn266->Thr | | | Cyanoacrylate 30 | Ketonitrife 0,4 |
| | | | Diuron 13 | |
| | | | Metamitron 50 | |
| | | | Metribuzin 1550 | |
| | | | Phenmedipham 1260 | |

| Deletion Mutants | | | | |
|---|---|---|---|---|
| ΔGlu229->Gln(Ala)233 | *Synechocystis sp. PC6803* | | Atrazine 1,5 | |
| | | | BNT 1 | |
| | | | Diuron 2,6 | |
| | | | loxynil 1,5 | |
| ΔAsn234->Gln236 | *Synechocystis sp. PC6803* | | Atrazine 5 | |
| | | | BNT 1,4 | |
| | | | Diuron 8,6 | |
| | | | loxynil 6 | |
| ΔTyr237->Phe239 | *Synechocystis* sp. *PC6803* | | Atrazine 6 | BNT 0,4 |
| | | | Diuron 23 | |
| | | | loxynil 4 | |
| △Gly240→Gln241 | *Synechocystis sp. PC6803* | | Atrazine 21 | BNT 0,5 |
| | | | Diuron 26 | |
| | | | loxynil 1,4 | |

| Deletion/insertion Mutant | | | | |
|---|---|---|---|---|
| △Ala250→Ala251 | *Synechocystis sp. PC6803* | | Atrazine 80 | |
| +Asn247→Tyr | | | BNT 2 | |
| | | | Diuron 280 | |
| | | | loxynil 5 | |

| | | | | |
|---|---|---|---|---|
| a) Arg in other organisms | | | | |
| b) Johanningmeier U, Sopp G, Brauner M, Altenfeld U, Orawski G, and Oettmeier W, to be published | | | | |
| c) denotes in the presence (+) or in the absence (-) of diuron at the given concentration in µM | | | | |

### Equipment

From an examination of the design problems for the fabrication of the sensor, the possible arrangements of the biosensor, from both modelling and experimental point of view, were considered in order to determinate the most suitable structure, the geometry of the system and the optoelectronic and chemical physical design parameters. Based on such specifications the measuring cell was designed, the components to be used and the operating procedure to be followed for analyzing of the herbicides by the biosensor were set up. The device is up to the selected transfer system.

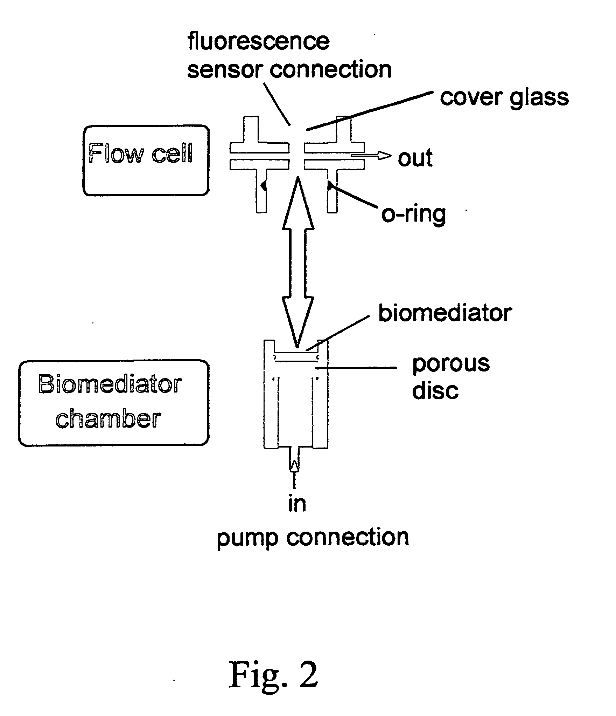

According to a specially advantageous embodiment, Bioherbi is an equipment weighing in all about 60 kg, occupies a space of about 30x30x20 cm and is constituted as reported below.
a) flow cell wherein the biomediator is immobilized. The diagram (see Fig. 2) shows schematically the cell which consists of black plastic material and has a diameter of about 4 cm. The black plastic material allows to fit the biomediator in the dark. The biomediator in entrapped in the inner section. The surface tightness of the reading hole is obtained using a glass cover plate for microscopy. The inner cell, where the mediator is entrapped, has un volume from 50 to 300 µl.
b) a peristaltic pump is the hydraulic part to withdraw the sample containing the herbicide to be analyzed, which moves said sample through the flow cell and brings it in close contact with the biomediator. According to a specially advantageous embodiment a single block is used as holder for the herbicide and peristaltic pump. The flow can be varied from 0,2 to 2 ml/min depending on the sample. Higher flow increases the sensitivity to the herbicide because the latter interacts with a higher number of bonding sites on PS II.
   By laboratory tests it was found out that, when the solution of the herbicide flows, the efficiency of the fluorescence measure could be impaired by light scattering effects. Accordingly it is preferred to carry out the measure in stopped-flow conditions using a flow switch.
   The biomediator immobilized within the flow cell, following each measure, is washed and regenerated by a suitable buffer solution. A valve allows to forward alternatively the flow of the buffer or the solution containing the herbicide to be analyzed.
c) optical fiber fluorescence sensor closely connected to the flow cell.
   The fluorescence emitted from green plants depends on the photosynthetic activity. Recent studies on fluorescence measuring techniques made this feature a very important tool in the basic and applied research about the plant physiology (D Lazar, 1999, Biochim Biophys Acta 1-28).
   Bioherbi analyzer measures the fluorescence emitted from photosynthetic complexes and modifications determined by the presence of herbicide.
   The sensor is tightly connected to the flow cell to avoid any infiltration of external light and provides the excitation light detecting the fluorescence signal. The excitation light is emitted from an emitting diode at a very narrow wavelength of about 650 nm, which is quickly absorbed by the Photosystem II of the photosynthetic organisms and has the advantage in that it provides light with reduced heating and emits the signal very quickly. A "feedback" optical circuit controls and adjusts the variations of the intensity of the emitted light also resulting from variations of ambient temperature. The analyzer consists of an high capacity photodiode associated to an amplifier. The optical system responds perfectly to the fluorescence emission wavelength of 700 nm and stops the reflection light of lower wavelength resulting from the emission light. The sensor is connected to the control computer by an about 30 cm long fiber.
   According to a variation of the biosensor we used a more complex fluorescence system, adjustable as the excitation light in order to allow the use, as biomediator, of photosynthetic organisms requiring specific wavelengths.
e) a control and data processing computer. The fluorescence key parameters, i.e. Fo, Fm, Fv and the Fv/Fm ratio, area over the fluorescence curve A (see Tables 1), the time Tm occurring to achieve maximum fluorescence, are automatically calculated and displayed on the liquid crystal monitor.
   The concentration of the herbicide is proportional to the area over the fluorescence curve (Table 1A-B); the inhibition of this area is closely related to the washing times whereby it is possible to determinate the concentration of the herbicide also using the washing time required to obtain an almost complete inhibition of the area (see Table 1C). The fluorescence signal received by the sensor is processed within the control chamber by transduction systems of the signal and analog-to-digital converters. The system is computerized with a very sophisticated microprocessor able to process the data of the fluorescence signal. The electronic system is therefore assembled in a portable device to make as easy as possible the in situ movements and allow the use by not specialized people. Furthermore the Bioherbi is equipped with a long service battery charger with internal protection of the data in case of battery discharging.
f) the system is equipped with a timer to measure automatically the fluorescence in stopped-flow conditions. The timer (0 - 60 min) enables at the same time both the system to stop the flow and allow the measure of the florescence in stopped-flow conditions and a delayer (0 - 60 sec) which, following the stopped-flow condition, enables the fluorescence pulse actuator.

### Advantages in comparison with other techniques and biosensors

Three techniques to detect the herbicides currently are used: HPLC, GC-MS and ELISA techniques, respectively. The HPLC and GC-MS techniques represent reliable routine methods but show the drawbacks in that require expensive equipments, remarkable amounts of both matrix to be analyzed and organic solvent, in turn potentially environmentally harmful. Furthermore it is necessary the treatment and pre-concentration of the matrix before the analysis thereof, thus limiting the number of daily analyses (Chimica analitica strumentale, Eds Skoog and Leary, Saunders College Publishing, Orlando, ISBN 88 7959 0669). A recently developed immunologic method (ELISA) has high sensitivity (2 x10⁻¹⁰ M for Diuron; 1 x 10⁻¹⁰ for atrazine) but requires the preparation of expensive monoclonal antibodies, which in turn must be prepared by using potentially harmful chemical compounds, and sacrifice of laboratory animals. Furthermore the monoclonal antibodies are specific for an individual compound and not for classes thereof, as more widely required (P Schneider, MH Goodrow, SJ Gee, BD Hammock, 1994, J Agricultural Food Chemistry, 42, 413-422).

In comparison with the prior biosensor systems (for a review on the subject see JL Marty, D Garcia, R Rouillon, Trends in Analytical Chemistry, 14, 329-332) the equipments object of this invention shows various advantages and originality:
- requires a minimal amount of photosynthetic organism (few micrograms as chlorophyll content);
- uses a biomediator immobilized and stable over the time;
- is regenerable following each measure within two days;
- combine more qualities: high sensitivity, specificity and easiness to be used;
- does not require highly specialized technical people;
- requires poor maintenance in comparison, for example, with the transduction system of the Clark electrode (M Koblizek, J Masojidek, J Komenda, T Kucera, R Pilloton, A Mattoo, MT Giardi, 1998, Biotechnology and Bioengineering, 60, 664-669), and, at the same sensitivity, does not require long time to reach the steady-state condition of the system;
- the object of present invention is surely easier to handle being a portable device weighing only 6 kg against 30 kg of the prior system (M Koblizek, J Masojidek, J Komenda, T Kucera, R Pilloton, A Mattoo, MT Giardi, 1998, Biotechnology and Bioengineering, 60, 664-669);
- the object of the invention is portable and computerized;
- it measure the area over the fluorescence curve whereas in previous biosensor systems a kinetics of fluorescence increasing in a period of seconds is carried out which requires a more complex and accurate measuring equipment (D Merz, M Geyer, M Moss, DA Anche, 1996, Fresenius J Analytical Chemistry 354, 299-305).

It is therefore a specific object of the invention a biomediator able to recognize and bind herbicides essentially consisting of: a) a first component comprising a Photosystem II protein complex and b) a second component consisting of a support, wherein the first component comprising the Photosystem II protein complex is immobilized on the support, said first component of the biomediator comprising the Photosystem II protein complex being advantageously pre-treated with a solution of heavy metal salts as, for example, cadmium or zinc salts and successively immobilized on the support.

Among the herbicides which can be detected by the biomediator according to the invention are those belonging to the following classes: ureidic, trazinic, diazinic and phenolic.

The first component of the biomediator comprising the Photosystem II protein complex can be constituted of cells from photosynthetic microorganisms or plants or extracts therefrom.

Among the photosynthetic microorganisms which can be used the preferred species are reported below: *Chlorella Sorokiniana, Chlorella Zofinngiensis, Spongiochloris Typea, Scenedesmus quadricauda, Koliella sp., Pleurochloris meringensis, Spirulina platensis, Synechoccoccus elongates.*

The plants which can be used for the purposes of the present invention are, for example, *Avena sativa, Pisum sativum, Poa annua, Senecio vulgaris, Solanum nigrum, Solanum Tuberosum, spinacea oleracea, Triticum durum, Vicia faba, Zea mays.*

A further object of the present invention is a biomediator wherein the microorganisms or the plants have a Photosystem II protein complex mutated in such a way to prove resistant to at least a subclass of herbicides.

The invention relates, in addition, to a biosensor for the environmental monitoring of herbicides comprising at least a biomediator according to before described and a detecting system as, for example, a fluorescence sensor.

The biosensor can include a series of two or more biomediators of the present invention and wherein at least one of said biomediator includes a Photosystem II protein complex mutated in such a way to prove resistant to at least a subclass of herbicides.

It is a further object of the invention a device for the herbicide selective environmental monitoring including: a) at least a flow cell wherein a biomediator according to before described is immobilized; b) a peristaltic pump to withdraw the sample containing the herbicide to be analyzed; c) a fluorescence sensor closely connected to each flow cell.; d) means for the control and processing of the data; e) and means for automatically measuring the fluorescence.

The invention relates, in addition, to a process for the preparation of a biomediator able to recognize and bind herbicides according to before described wherein the component comprising the Photosystem II protein complex is treated with a solution of heavy metal salts as, for example, cadmium or zinc salts and successively immobilized on the support.

It is a further object of the invention a method for the detection of the presence of herbicides in a sample, comprising the following steps:
a) exposure of the sample to one or more biomediators according to before described wherein each biomediator is contained in a flow cell;
b) connection of the flow cells with a fluorescence detecting system;
c) detection of the fluorescence signal;
d) removal of the sample and wash and regeneration of the biomediator.

The biomediators used in said method can be two or more and can be arranged in series or parallel.

Furthermore at least one of the biomediators can include a Photosystem II protein complex mutated in such a way to prove resistant to at least a subclass of herbicides.

The present invention now will be described by explaining but non limiting way according to some embodiments thereof with particular reference to the figures of the appended drawings wherein:
Figure 1 shows a block diagram of the device for the herbicide selective monitoring according to the invention.
Figure 2 shows the structure of the filtering cell of the device for the herbicide selective monitoring according to the invention.
Figure 3 shows a photograph of the device according to the invention.
Figure 4 shows the possible arrangements of the biomediators and fluorescence detectors in the device according to the invention: (A) biomediators in series and interchangeable detector; (B) biomediators in parallel, each coupled with a fluorescence detector.

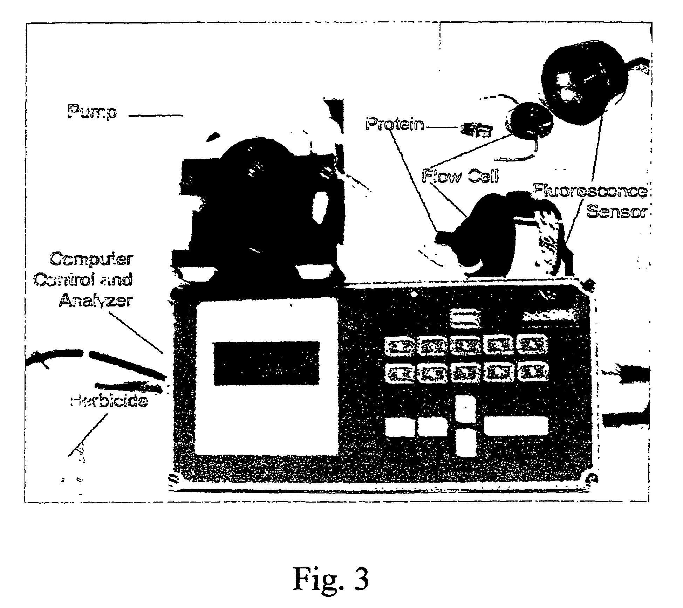

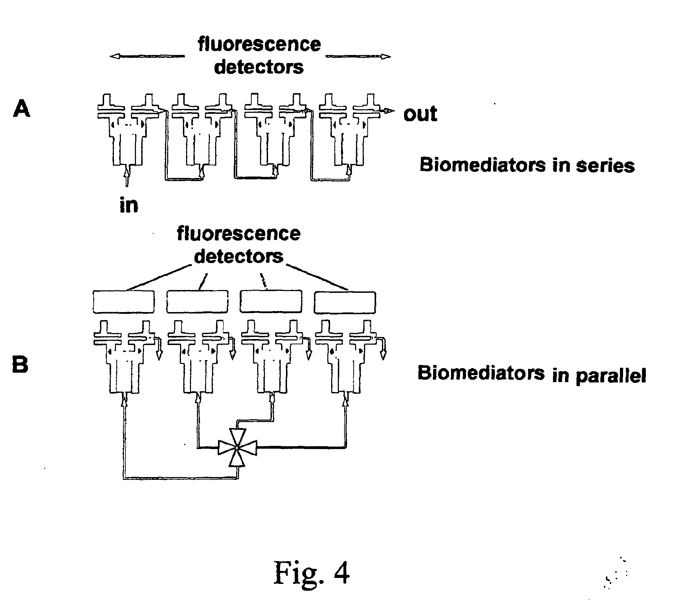

The invention will be understood more completely with reference to the description and the enclosed photograph (Fig. 3). It is understood that Figure 3 shows an embodiment given only as practical example of the invention, being possible to carry out variations and modifications as for the shape and arrangement depending on the immobilization type used for the biomediator, without departing, anyway, from the scope and the spirit of the invention. There are possible various arrangement of the Bioherbi which can involve the use sequentially or simultaneously of different biomediators immobilized and sensitive to different herbicide subclasses. For example, following a positive response in the presence of herbicides (see for example Table D) the body containing the biomediator is replaced by other bodies containing biomediators immobilized, specific and resistant to different subclasses. In the case shown in Figure 4A various measuring cells, each containing a specific immobilized biomediator, are arranged in series. The solution to be analyzed containing the herbicide flows through the cells whereas the fluorescence detector is moved from a cell to the next.

Whereas in Figure 4B various measuring cells, each containing an immobilized specific biomediator, are arranged in parallel. The solution to be analyzed, containing the herbicide, flows through a multi-way valve feeding individually the detecting cells and, after a positive response at the "wild-type" biomediator, automatically drives the flow into the next cell for the identification of the subclasses.

The obtained results allows to have a practical use of such a biosensor system based on PS II; in fact the technologies for the environmental analyses, which at the same time provide the measure and do not result in pollution, meet better the ever increasing restrictive specifications.

### Example 1: Preparation of PS II particles from plants

The following species were used: *Poa Annua* (poa), *Avena sativa* (avena), *Pisum sativum, Spinacea oleracea* (spinach), *Solanum Nigrum* (solano), *Senecio vulgaris* (senecio), *Solanum tuberosum* (potato), *Triticum durum* (wheat), *Vicia faba* (broad bean), *Zea mays* (mais) and the atrazine resistant mutants *Solanum nigrum* and *Senecio vulgaris* obtained from wild-type strain by plant selection after long-term treatment with atrazine. Seedlings were grown in soil or in vermiculite in plastic containers and watered with Hoagland's solution. The greenhouse temperature was maintained at 25°C with a growth irradiance of 450 µmol photons m⁻²s⁻¹. Mature plants were used for isolation of thylakoids and of PS II particles by the method of Berthold et al. (1981) using Triton X-100/chlorophyll ratio of 15 or 20 (Giardi et al., 1994). Chlorophyll content was determined in 80 % (v/v) acetone extracts of the tissue (Lichtenthaler and Wellburn, 1983).

### Example 2: Preparation of PS II particles from cyanobacteria

A continuous culture of the thermophilic cyanobacterium *Synechococcus elongatus* (30-40 mgg Chl/L) was grown at 56°C. Spheroplasts were prepared by lysozyme treatment of cells and, following an osmotic shock, thylakoid membranes were collected by centrifugation. The PS II particles from thylakoids were obtained by using either a zwitterionic detergent sulfobetain 12 (SB12) and a detergent to chlorophyll ratio of 3,5 or a non ionic detergent heptylthioglucoside (HGT) and a detergent to chlorophyll ratio of 8. Following the addition of 10 % detergent solution, the membranes were gently stirred for 20 min at 4°C and then centrifuged at 250000 x g for 30 min. The PS II particles solubilized by this procedure were frozen in liquid nitrogen and stored at -75°C. The chlorophyll content was determined in methanol extracts of the particles (Lichtenthaler and Wellburn, 1983).

### Example 3: Preparation of PS II particles from algae

Thylakoids from algae were prepared by washing the cells with a solution containing sodium phosphate buffer, pH 7,8 (10 mM), MgCl₂ (1 mM), BSA (0,5 %), EDTA (5 mM), PMSF (1 mM) and p-aminobenzamidine (1 mM) and resuspended in the same medium with additional 0,3 M sucrose. The cells were broken by French-press treatment at 500 bar and differentially centrifuged, first for 2,5 min at 300 x g and then the supernatant for 20 min at 10000 x g. The resulting membrane pellet was resuspended in a solution in a solution of Tricine-NaOH, pH 7,8 (10 mM), MgCl₂ (1 mM), NaCl (10 mM) and sucrose (0,3 M) and adjusted to a chlorophyll content of 1 mg/ml. Subsequently thylakoids were used for SDS polyacrylamide gel electrophoresis (17,5 % acrylamide) and Westem blotting (Sandmann, Kuhn & Boger, 1993 *Photosynthesis Research* vol:35: 185-190).

### Example 4: Biosensor response to herbicides as a function of the biomediator and contact times.

Table (A) shows the response of the device using the biomediator which includes *Vicia faba* thylakoids immobilized on porous filter (G3) and extracted by a buffer containing 0,4 % BSA. The contact time was 20 min under the flow of herbicide containing solution. The reported values are a mean of at least three measure (SE about 15 %). The % inhibition value was obtained from the ratio of the difference between the control and in the presence of herbicide areas to the area obtained for the control, expressed in per cent.

Table (B) shows the response of the device using the biomediator based on thylakoids from *Spinacia oleracea.* Contact time of 15 min under the flow of herbicide containing solution.

Table (C) shows the response of the device using the biomediator based on thylakoids from *Spinacia oleracea.* Measures carried out at successive times under the flow of herbicide containing solution. The measures are an average from at least three analyses, ES about 10 %.

Table (D) shows the % inhibitions obtained using a biomediator based on thylakoids from Amaranthus retroflexus, herbicide concentration of 10⁻⁶ M. The measure are an average from at least three analyses, ES about 16 %.

**Table A**

| | Fv/Fm | Area | % Inhibition |
|---|---|---|---|
| Control | 0,694 | 1037 | 0 |
| Diuron 10⁻⁶ M | 0,540 | 10300 | 93 |
| Regeneration (10 min - CdCl₂) | 0,622 | 720 | / |
| Diuron 10⁻⁹ M | 0,475 | 4030 | 60 |

**Table B**

| | Control | Diuron 10⁻⁸ M | Diuron 10⁻⁸ M |
|---|---|---|---|
| Fv/Fm | 0,778 | 0,768 | 0,756 |
| Area | 14500 | 12700 | 10400 |
| % Inhibition | 100 | 88 | 71 |

**Table C**

| | Fv/Fm | Area | % Inhibition |
|---|---|---|---|
| Control | 0,755 | 11500 | 0 |
| Diuron (10⁻⁷M) 10 min | 0,746 | 7830 | 32 |
| Diuron (10⁻⁷M) 40 min | 0,711 | 6120 | 47 |
| Diuron (10⁻⁷M) 50 min | 0,700 | 5090 | 56 |
| Diuron (10⁻⁷M) 60 min | 0,684 | 4160 | 64 |

**Table D**

| Area % inhibition | | |
|---|---|---|
| | Wild-type | Mutant |
| Diuron | 99 | 96 |
| Bromoxynil | 97 | 95 |
| Dinoseb | 99 | 93 |
| Atrazine | 96 | 15 |
| Simazine | 95 | 14 |
| Ametrine | 99 | 21 |
| Desmetryn | 92 | 10 |

## Claims

1. Biomediator able to recognize and bind herbicide consisting of: a) a first component comprising a Photosystem II protein complex and b) a second component consisting of a support, wherein the first component comprising the Photosystem II protein complex is immobilized on the support, said first component comprising the Photosystem II protein complex being pre-treated with a solution of heavy metal salts and subsequently immobilized on the support.

2. Biomediator according to claim 1, wherein the heavy metals are cadmium or zinc.

3. Biomediator according to claim 1, wherein the herbicides belong to the following classes: ureidic, triazinic, diazinic, phenolic.

4. Biomediator according to anyone of the preceding claims, wherein the first component comprising a Photosystem II protein complex consists of cells from photosynthetic microrganisms or plants or extracts therefrom.

5. Biomediator according to claim 4, wherein the photosynthetic microrganisms are selected from the group consisting of the following species: *Chlorella Sorokiniana, Chlorella Zofinngiensis, Spongiochloris Typea, Scenedesmus quadricauda, Koliella sp., Pleurochloris meringensis, Spirulina platensis, Synechoccoccus elongates.*

6. Biomediator according to claim 4, wherein the plants are selected from the group consisting of Avena sativa, Pisum sativum, Poa annua, Senecio vulgaris, Solanum nigrum, Solanum Tuberosum, spinacea oleracea, Triticum durum, Vicia faba, Zea mays.

7. Biomediator according to anyone of the preceding claims wherein the microorganisms or plants have a Photosystem II protein complex mutated in such a way to prove resistant to at least a subclass of herbicides.

8. Biosensor for the environmental monitoring of herbicides comprising at least a biomediator according to anyone of claims 1-7 and a detecting system.

9. Biosensor according to claim 8, wherein the detecting system is a fluorescence sensor.

10. Biosensor according to claim 8 or 9 comprising a series of two or more biomediators according to anyone of claims 1-7 and wherein at least one of said biomediators comprises a Photosystem II protein complex mutated in such a way to prove resistant to at least a subclass of herbicides.

11. Device for the herbicide selective environmental monitoring including: a) at least a flow cell wherein a biomediator according to claims 1-7 is immobilized; b) a peristaltic pump to withdraw the sample containing the herbicide to be analyzed; c) a fluorescence sensor connected to each flow cell.; d) means for the control and processing of the data; e) and means for measuring automatically the fluorescence.

12. Process for the preparation of a biomediator able to recognize and bind herbicides according to claim 1, wherein the component comprising the Photosystem II protein complex is treated with a solution of heavy metals as, for example, cadmium or zinc salts and successively immobilized on the support.

13. Process according to claim 12, wherein the heavy metal salt is cadmium chloride.

14. Method for the detection of the presence of herbicides in a sample, comprising the following steps:
a) exposure of the sample to one ore more biomediators according to anyone of claims 1-7 wherein each biomediator is contained in a flow cell;
b) connection of the flow cells with a fluorescence detecting system;
c) detection of the fluorescence signal;
d) removal of the sample and wash and regeneration of the biomediator.

15. Method for the detection of the presence of herbicides in a sample according to claim 14, wherein the biomediators are two or more and are arranged in series or parallel.

16. Method for the detection of the presence of herbicides in a sample according to claim 14 or 15, wherein at least one the biomediators includes the Photosystem II protein complex mutated in such a way to prove resistant to at least a subclass of herbicides.

## Patentansprüche

1. Biomediator, der ein Herbizid erkennen und binden kann, bestehend aus: (a) einer ersten Komponente, umfassend einen Fotosystem II-Protein-Komplex, und (b) einer zweiten Komponente, bestehend aus einem Träger, wobei die erste Komponente, umfassend den Fotosystem II-Protein-Komplex, auf dem Träger immobilisiert ist, wobei die erste Komponente, umfassend den Fotosystem II-Protein-Komplex, mit einer Lösung von Schwermetallsalzen vorbehandelt und darauffolgend auf dem Träger immobilisiert ist.

2. Biomediator gemäss Anspruch 1, wobei die Schwermetalle Kadmium oder Zink sind.

3. Biomediator gemäss Anspruch 1, wobei die Herbizide zu den folgenden Klassen gehören: Ureido-, Triazin-, Diazin-, phenolisch.

4. Biomediator gemäss einem der vorstehenden Ansprüche, wobei die erste Komponente, umfassend einen Fotosystem II-Protein-Komplex, aus Zellen von fotosynthetischen Mikroorganismen oder Pflanzen oder Extrakten daraus besteht.

5. Biomediator gemäss Anspruch 4, wobei die fotosynthetischen Mikroorganismen gewählt sind aus der Gruppe, bestehend aus den folgenden Arten: Chlorella sorokiniana, Chlorella zofingiensis, Spongiochloris typea, Scenedesmus quadriacauda, Koliella sp., Pleurochloris meringensis, Spirulina platensis, Synechoccoccus elongates.

6. Biomediator gemäss Anspruch 4, wobei die Pflanzen gewählt sind aus der Gruppe, bestehend aus Avena sativa, Pisum sativum, Poa annua, Senecio vulgaris, Solanum nigrum, Solanum tuberosum, Spinacea oleracea, Triticum durum, Vicia faba, Zea mays.

7. Biomediator gemäss einem der vorstehenden Ansprüche, wobei die Mikroorganismen oder Pflanzen einen Fotosystem II-Protein-Komplex aufweisen, der derart mutiert ist, dass er sich gegenüber mindestens einer Subklasse von Herbiziden als resistent erweist.

8. Biosensor für die Umweltüberwachung von Herbiziden, umfassend mindestens einen Biomediator gemäss einem der Ansprüche 1 bis 7 und ein Nachweissystem.

9. Biosensor gemäss Anspruch 8, wobei das Nachweissystem ein Fluoreszenzsensor ist.

10. Biosensor gemäss Anspruch 8 oder 9, umfassend eine Reihe von zwei oder mehr Biomediatoren gemäss einem der Ansprüche 1 bis 7, und wobei mindestens einer der Biomediatoren einen Fotosystem II-Protein-Komplex umfasst, der derart mutiert ist, dass er sich als gegenüber mindestens einer Subklasse von Herbiziden resistent erweist.

11. Vorrichtung für eine herbizid selektive Umweltüberwachung, beinhaltend: (a) mindestens eine Flusszelle, worin ein Biomediator gemäss den Ansprüchen 1 bis 7 immobilisiert ist; (b) eine peristaltische Pumpe, um die Probe zu entnehmen, enthaltend das zu analysierende Herbizid; (c) einen Fluoreszenzsensor, verbunden mit jeder Flusszelle; (d) Datenkontroll- und -verarbeitungsmittel; und (e) Mittel zur automatischen Messung der Fluoreszenz.

12. Verfahren für die Herstellung eines Biomediators, der Herbizide erkennen und binden kann, gemäss Anspruch 1, wobei die Komponente, umfassend den Fotosystem II-Protein-Komplex, mit einer Lösung aus Schwermetallen, wie z.B. Kadmium- oder Zinksalzen, behandelt ist und darauffolgend auf dem Träger immobilisiert wird.

13. Verfahren gemäss Anspruch 12, wobei das Schwermetallsalz Kadmiumchlorid ist.

14. Nachweisverfahren für die Gegenwart von Herbiziden in einer Probe, umfassend die folgenden Schritte:
(a) Aussetzen der Probe gegenüber einem oder mehreren Biomediatoren gemäss einem der Ansprüche 1 bis 7, wobei jeder Biomediator in einer Flusszelle enthalten ist;
(b) Verbinden der Flusszelle mit einem Fluoreszenz-Nachweissystem;
(c) Nachweisen des Fluoreszenzsignals;
(d) Entfernen der Probe und Waschen und Regenerieren des Biomediators.

15. Nachweisverfahren für die Gegenwart von Herbiziden in einer. Probe gemäss Anspruch 14, wobei die Biomediatoren zwei oder mehr sind und in Reihe oder parallel angeordnet sind.

16. Nachweisverfahren für die Gegenwart von Herbiziden in einer Probe gemäss Anspruch 14 oder 15, worin mindestens einer der Biomediatoren den Fotosystem II-Protein-Komplex derart mutiert beinhaltet, dass er sich als resistent gegenüber mindestens einer Subklasse von Herbiziden erweist.

## Revendications

1. Biomédiateur capable de reconnaître et de fixer un herbicide comprenant : a) un premier composant comportant un complexe de protéine de photosystème II et b) un deuxième composant comportant un support, dans lequel le premier composant comprenant le complexe de protéine de photosystème II est immobilisé sur le support, ledit premier composant comprenant le complexe de protéine de photosystème II étant prétraité avec une solution de sels de métaux lourds et subséquemment immobilisé sur le support.

2. Biomédiateur selon la revendication 1, dans lequel les métaux lourds sont le cadmium ou le zinc.

3. Biomédiateur selon la revendication 1, dans lequel les herbicides appartiennent aux classes suivantes : uréidique, triazinique, diazinique, phénolique.

4. Biomédiateur selon l'une quelconque des revendications précédentes, dans lequel le premier composant comportant un complexe de protéine de photosystème II comprend des cellules de micro-organismes photosynthétiques ou de plantes ou d'extraits de celles-ci.

5. Biomédiateur selon la revendication 4, dans lequel les micro-organismes photosynthétiques sont choisis dans le groupe comprenant les espèces suivantes : Chlorella Sorokiniana, Chorella Zofinngiensis, Spongiochloris Typea, Scenedesmus quadricauda, Koliella sp., Pleurochloris meringensis, Spirulina platensis, Synechoccoccus elongates.

6. Biomédiateur selon la revendication 4, dans lequel les plantes sont choisies dans le groupe comprenant Avena sativa, Pisum sativum, Poa annua, Senecio vulgaris, Solanum nigrum, Solanum Tuberosum, spinacea oleracea, Triticum durum,Vicia faba, Zea mays.

7. Biomédiateur selon l'une quelconque des revendications précédentes, dans lequel les micro-organismes ou les plantes ont un complexe de protéine de photosystème II muté de telle façon à être résistant à au moins une sous-classe d'herbicides.

8. Biocapteur pour le contrôle environnemental d'herbicides comprenant au moins un biomédiateur selon l'une quelconque des revendications 1 à 7 et un système de détection.

9. Biocapteur selon la revendication 8, dans lequel le système de détection est un capteur de fluorescence.

10. Biocapteur selon la revendication 8 ou 9, comprenant une série de deux ou plusieurs biomédiateurs selon l'une quelconque des revendications 1 à 7 et dans lequel au moins l'un desdits biomédiateurs comprend un complexe de protéine de photosystème II muté de façon à être résistant à.au moins une sous-classe d'herbicides.

11. Dispositif pour le contrôle environnemental sélectif d'herbicides comprenant : a) au moins une cellule à écoulement dans laquelle un biomédiateur selon l'une quelconque des revendications 1 à 7 est immobilisé ; b) une pompe péristaltique pour retirer l'échantillon contenant l'herbicide à analyser; c) un capteur de fluorescence relié à chaque cellule à écoulement ; d) um moyen pour le contrôle et le traitement des données ; e) et des moyens pour mesurer automatiquement la fluorescence.

12. Procédé de préparation d'un biomédiateur capable de reconnaître et de fixer les herbicides selon la revendication 1, dans lequel le composant comprenant le complexe de protéine de photosystème II est traité avec une solution de métaux lourds comme, par exemple, des sels de cadmium ou de zinc et immobilisé successivement sur le support.

13. Procédé selon la revendication 12, dans lequel le sel de métal lourd est du chlorure de cadmium.

14. Procédé pour la détection de la présence d'herbicides dans un échantillon, comprenant les étapes suivantes :
a) l'exposition de l'échantillon à un ou plusieurs biomédiateurs selon l'une quelconque des revendications 1 à 7, dans lequel chaque biomédiateur est contenu dans une cellule à écoulement ;
b) la liaison des cellules à écoulement à un système de détection de fluorescence ;
c) la détection du signal de fluorescence ;
d) l'élimination de l'échantillon et le lavage et la régénération du biomédiateur.

15. Procédé pour la détection de la présence d'herbicides dans un échantillon selon la revendication 14, dans lequel les biomédiateurs sont deux ou plusieurs et sont agencés en série ou en parallèle.

16. Procédé pour la détection de la présence d'herbicides dans un échantillon selon la revendication 14 ou 15, dans lequel au moins l'un des biomédiateurs comprend le complexe de protéine de photosystème II muté de façon à être résistant au moins à une sous-classe d'herbicides.
